(19) Europäisches Patentamt
European Patent Office
Office européen des brevets

(11) Publication number: **0 191 236 A1**

# (12) EUROPEAN PATENT APPLICATION

(21) Application number: 85309162.7

(22) Date of filing: 16.12.85

(51) Int. Cl.[4]: **A01N 43/42**, A01N 43/40, A01N 33/12

(30) Priority: 16.01.85 IL 74067

(43) Date of publication of application: 20.08.86 Bulletin 86/34

(84) Designated Contracting States: AT BE CH DE FR GB IT LI LU NL SE

(71) Applicant: ATARON DEVELOPMENT (1985) LTD.
Atarot Industrial Center P.O. Box 1365
91013 Jerusalem(IL)

(72) Inventor: Konis, Yoel, Dr.
23/72 Golomb Street
Jerusalem(IL)

(74) Representative: Knott, Stephen Gilbert et al
MATHISEN, MACARA & CO. The Coach House 6-8 Swakeleys Road
Ickenham Uxbridge Middlesex UB10 8BZ(GB)

## (54) Insecticidal compositions.

(57) The invention provides insecticidal and especially pediculocidal compositions comprising a quaternary ammonium compound of the general formula I

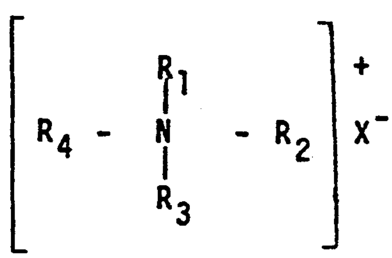

I

wherein each of $R_1$, $R_2$, $R_3$ and $R_4$ may be the same or different and are each individually selected from the group consisting of a $C_1$-$C_{20}$ alkyl group or a benzyl group or wherein $R_2$, $R_3$ and $R_4$ are combined with the N to which they are attached to form an optionally substituted pyridine or quinoline group and X is halogen.

EP 0 191 236 A1

# INSECTICIDAL COMPOSITIONS

The present invention relates to insecticidal compositions, and in particular to pediculocidal compositions.

The term pediculocidal as used herein is intended to refer to a composition useful in the treatment of head and/or body and/or crab lice and/or lice eggs of all kinds.

Hardly a city or village exists in any part of the world where not one or more individuals are infested with head lice. In the U.S.A. alone there were approximately 3 million known cases of Pediculosis in 1974.

The present lice infestation is treated by either a mechanical procedure (in combing the hair with a special comb, or pulling the lice (body lice) from one's clothes, or by use of insecticides. The insecticides, commonly used for lice treatment could be divided into two categories:

1. Insecticides with relatively low toxicity.

2. Insecticides with relatively high toxicity.

The latter are more effective, but usually are given only under physicians' prescriptions. All these substances are supplied as lotions, shampoos, creams, dust or sprays.

There is an almost countless number of insecticides that have been developed during the last 40-50 years, but only part of them are in common use (world wide) today. According to a report, published in 1982 by the United Nations World Health Organization (WHO), there are only 10 compounds in everyday use (table 1). Three of the most commonly used are: DDT, Lindane, Malathion.

TABLE 1

| Insecticide Type | Chemical | Formulation | Concentration % | Oral LD 50 to rats (mg/kg) |
|---|---|---|---|---|
| Bioallethrin (+ allethrin) | Py | Lotion | 0.3-0.4 | |
| | | Shampoo | 0.3-0.4 | 500 |
| | | Aerosol | 0.6 | |
| Carbaryl | C | Dust | 5.0 | 300 |
| DDT | OC | Dust | 10.0 | 113 |
| | | Lotion | 2.0 | |
| Dellamethrin | Py | Lotion | 0.03 | |
| | | Shampoo | 0.03 | 135 |
| Jodfenphos | OP | Dust | 5.0 | 2100 |
| Lindane (λ-HCH) | OC | Dust | 1.0 | 100 |
| | | Lotion | 1.0 | |
| Malathion | OP | Dust | 1.0 | 2100 |
| | | Lotion | 0.5 | |
| Permethrin ( + Pyrethrin) | Py | Dust | 0.5 | |
| | | Lotion | 1.0 | 4000 |
| | | Shampoo | 1.0 | |
| Propoxur | C | Dust | 5.0 | 95 |
| Temephos | OP | Dust | 2.0 | 8600 |

Those insecticides can be grouped into 4 groups, according to their chemical structure:

1. Organochlorines (O.C.)

2. Organophosphates (O.P.)

3. Carbamattes (C.)

4. Pyrethroids and synthetic pyrethroids.

The first 3 groups are highly toxic, whilst the fourth group has low toxicity - but it is not very effective.

It is quite clear from data being received so far, that insecticides are all toxic and not at a low degree. However, there are more disadvantages in their use than that. Some of them are not very effective in killing eggs (Pyrethrin, bioallethin), according to Lange K. et al, Acta Dermatovener, 61; 91-92 (1980)-Shovt Common; Carbaryl, Lyndane and DDT according to Maunder J.W. Clinical and Experimental Dermatology. 6; 605-612 (1981); or must be used in high concentrations to achieve their purpose. Others are not very effective in adults and nymphs (Dieldrin and Lindane according to Bloomers L., J. Med. Entemol. 16;

82-83 (1979). Lindane is also being suspected of being a carcinogenic compound (Review by IARC; Schacter B.J. of the Amer. Acad. of Derm. 5; 517-527 (1981). Acquired resistance developed by the lice against some of the compounds is also quite common these days. It was first reported by Hurlbut H.S. et al Science. 115; 11-12 (1952) who found DDT resistance in body lice. Later other compounds were found to be less effective because of resistancy, such as Lindane and Malathion (Maunder J.W. Medical officer 125; 27-29 (1971); Wright J.W. et al, Bull. Wld. Hlth. Org. 33; 485-501 (1955); Miller R.N. et al, Trans. Roy. Soc. Trop. Med. Hyg. 66; 372-375 (1972).

In conclusion - the compounds at present in use are having disadvantages on three levels:

1. They are toxic.

2. They are not very effective against eggs.

3. The lice developed resistancy against some of them.

According to the present invention it has now been surprisingly discovered that quaternary ammonium compounds which have been used in many different non-related applications and which are considered to be relatively nontoxic can be effectively used in pediculocidal compositions.

Since lice are a form of insect and are related to such other insects as termites, it is believed that the present composition can also be properly characterized as insecticidal compositions and also used as such.

Thus, the present invention provides an insecticidal composition characterized in that it comprises a quaternary ammonium compound of the general formula I

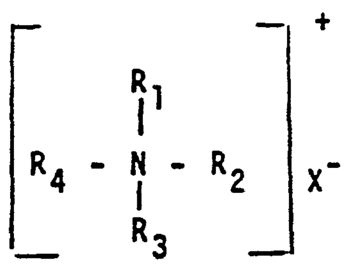

I

wherein each of $R_1$, $R_2$, $R_3$ and $R_4$ may be the same or different and are each individually selected from the group consisting of a $C_1$-$C_{20}$ alkyl group or a benzyl group or wherein $R_2$, $R_3$ and $R_4$ are combined with the N to which they are attached to form an optionally substituted pyridine or quinoline group and X is halogen.

In especially preferred compositions, at least one of $R_1$, $R_2$, $R_3$ and $R_4$ is an alkyl group and preferably at least one of $R_1$, $R_2$, $R_3$ and $R_4$ is an alkyl group having 8 to 18 carbon atoms.

As indicated hereinbefore, the compositions of the present invention have been found to be particularly useful as anti-lice compounds and thus in a preferred embodiment of the present invention there is provided a pediculocidal composition comprising a cosmetically acceptable quarternary ammonium compound of the general formula I wherein $R_1$, $R_2$, $R_3$ and $R_4$ may be the same or different and are each individually selected from the group consisting of methyl, $C_8$ to $C_{18}$ alkyl, or benzyl or wherein $R_2$, $R_3$ and $R_4$ are combined with the N to which they are attached to form an optionally substituted pyridine or quinoline group.

Preferred compositions are those wherein at least one of $R_1$, $R_2$, $R_3$ or $R_4$ is an alkyl group having 8 to 18 carbon atoms and especially preferred are compositions wherein at least one of $R_1$, $R_2$, $R_3$ or $R_4$ is selected from the group consisting of methyl, cetyl, lauryl or benzyl.

In the above compounds X is preferably iodine, bromine or chlorine and chlorine is especially preferred.

The compositions of the present invention will preferably contain between about 0.8 and about 8.0% w/v of the quaternary ammonium compound of formula I as active ingredient therein, although smaller amounts might also be used in certain cases, and especially preferred are compositions containing between about 1 and 4% w/v active ingredient.

The compositions of the present invention can be administered as dry powders or as aqueous or alcohol solutions and preferred compositions will contain about 0 to 70% of an alcohol solvent wherein said alcohol is preferably selected from ethanol, isopropanol and mixtures thereof.

In summary, as is known, quaternary ammonium compounds (hereinafter referred to as QACS) are derivates of amines. They are made up by the gentle warming of tetriary amines plus alkyl halide (as carbonium ion) in alcoholic solvent - resulting the percipitation of the QAC as crystaline salt. Since the nitrogen atom of the molecule is tied to 4 alkyl chains ("R"), optical isomers are formed. Any "R" chain can be made of almost any alkyl exists (within some limits) like: benzens, methyl, ethyl, cetyl, lauryl, oleyl, any fatty acid, etc.

Of the many hundreds QAC known so far about 90 are allowed to be used in the cosmetic industry. Probably all these compounds are, to some extent, lethal to any lice colony (especially at higher concentrations) since all of the quaternary ammonium salts tested caused some mortality, but there are preferable compounds.

QACS are characterized by a very large cation and a small anion - mostly a hologen. In general, QACS containing $Cl^-$ (as anion) are more active (as lice killers) than those containing $Br^-$, and those containing $Br^-$ are more active than those with $I^-$ or phosphate anion.

The structure of the cation is also of importance. It has been noted that a cation containing a "dimethyl" group is more lethal to the lice than one containing a "trimethyl" group. The remaining R/R's is best to be 8-18 carbons chain, preferably Lauryl-$CH_3(CH_2)_{11}$; Cetyl - $CH_3(CH_2)_{15}$ or Benzyl- 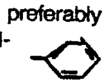 groups. The Pyridinium cation

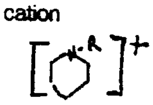

was however also found to be very active.

Quaternary ammonium compounds are also known as cationic detergents. Many modern germicides, fungicides and preservatives are quaternary ammonium compounds, the most well known (in use) is probably Benzalkonium chloride, which serves as sterilizing agent, but other, as cetyl pyridinum chloride or cetalkonium chloride serve the same purposes.

In general, all quaternary ammonium compounds are combined of a very big cation of 1 to 4 Carbon Chains surrounding the N molecule and a small anion - mostly a halogen: Chloride, Bromide, Iodine - but other anions as well. They are all good surfactants.

Quaternary ammonium compounds also serve as anti static agents in hair conditioners and hair tonics. In restaurants, dairies, etc. they are popular disinfectants for containers, instruments, etc. In medicine, diluted solutions have been employed to sterilize the skin, conjunctivae and mucos membranes. They have also been used to keep water pools free of slime, mould, algae, fish pathogens and molluskas. Some served in textile industry as moth proofing agents, others as oil preservatives, soil pathogen eradicators, foilage sprays and deodorants. They behave like acidic colorants in textile.

The concentrations usually used for disinfection, etc. are between 0.01-1.0%. The lethal effect, however, of quaternary ammonium compounds on lice, i.e., their action as "insecticides", had never been mentioned in the literature.

The preferred compounds for use in the compositions of the present invention are:

1. Cetyl trimethyl ammonium chloride

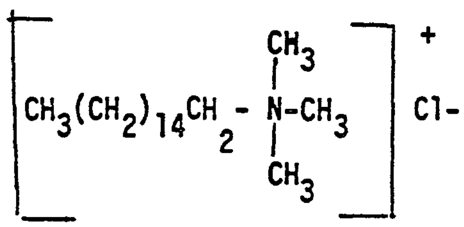

2. Lauryl dimethyl Benzyl ammonium chloride

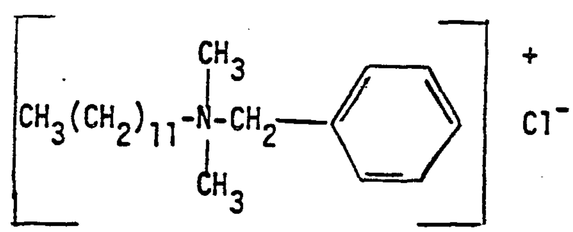

3. Lauryl Pyridinium chloride

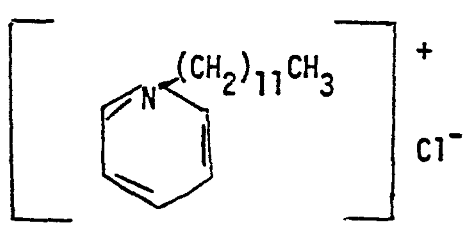

4. Tetra methyl ammonium bromide

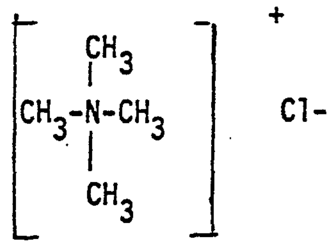

5. Lauryl isoquinolinium bromide

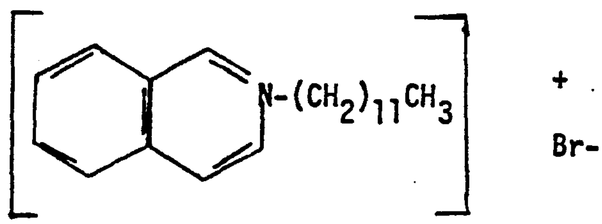

6. N-(lauryl Colamino Formyl Methyl) Pyridinium chloride

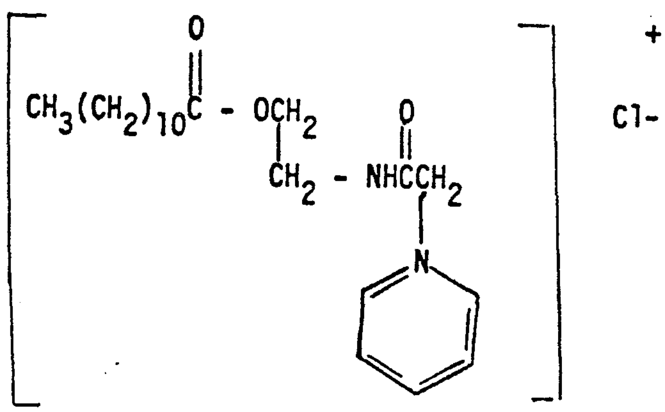

7. Lauralkonium bromide

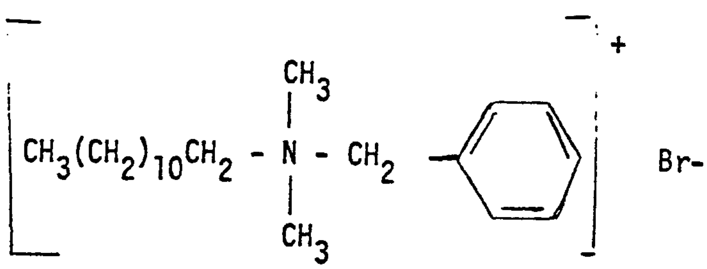

8. Cetyl trimethyl ammonium bromide

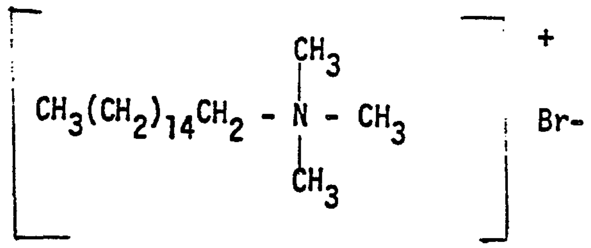

The most preferred compound is lauryl dimethyl benzyl ammonium chloride and the second best compound is cetyl trimethyl ammonium chloride.

Lauryl pyridinium chloride is also especially preferred since it can be applied as a solid.

As mentioned before, anti lice products can be used in various ways, of which the common ones are shampoos, dusts, sprays, creams and lotions. Since we are dealing with cationic detergents, using them as shampoos (mixed with animonic detergent) is not possible, whilst the other kinds are feasible. Since sprays are not very effective anyway, powders ("Dust") are limited only to dry compounds, such as Lauryl pyridinium chloride, the formula possible would be 15-10% Lauryl pyridinium chloride in a filler base as China clay.

The recommended way of using the compounds would be as alcoholic or watery lotions, such as:

A. <u>Alcoholic anti lice lotion: (w/v)</u>

40-60% Ethanol

0-10% Iso-propanol

0.8-8.0% Active ingredient

0-0.3% Mint

q,s.b. Color

q.s.b. Fragrance

add water up to 100.0

B. <u>Watery anti lice lotion: (w/v)</u>

0-40% Ethanol

0-10% Iso-propanol

0.8-8.0% Active ingredient

0-0.3% Mint

q.s.b. Color

q.s.b. Fragrance

add water up to 100.0

A spray would be based on the alcoholic formula, plus inertic gas such as freon.

Cream can also be produced by using an o/w or w/o cream, containing 0.8-4% active ingredient as part of the water phase.

While the invention will now be described in connection with certain preferred embodiments in the following examples, it will be understood that it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars described are by way of example and for purposes of illustrative discussion of preferred embodiments of the present invention only and are presented in the cause of providing what is believed to be the most useful and readily understood description of procedures as well as of the principles and conceptual aspects of the invention.

Example 1 - Lauryl pyridinium chloride

The following formulation was prepared by dissolving the lauryl pyridinium chloride in water with mild heating followed by cooking and addition of the alcohols:

45% Ethanol

10% Iso-propanol

2% Lauryl pyridinium chloride

43% Water deionized

EXAMPLE 2 - Lauryl dimethyl ammonium chloride

Since lauryl dimethyl ammonium chloride is liquid there is no need for dissolving in water with heating as in Example 1 and therefore, the following two formulations were prepared by simple mixing of all the ingredients together:

a) 46% Ethanol

10% Isopropanol

1% lauryl dimethyl ammonium chloride

43% deionized water

b) 56% Ethanol

1% lauryl dimethyl ammonium chloride

43% deionized water

EXAMPLE 3 - Cetyl trimethyl ammonium chloride

Following the procedure of Example 2, the following two formulations were prepared:

a) 46% Ethanol

10% Isopropanol

1% cetyl trimethyl ammonium chloride

43% deionized water

b) 56% Ethanol

1% cetyl trimethyl ammonium chloride

43% deionized water

EXAMPLE 4 - Tetramethyl ammonium bromide

Following the procedure of Example 2, the following two formulations were prepared:

a) 43% ethanol

12% isopropanol

4% tetramethyl ammonium bromide

46% deionized water

b) 50% ethanol

4% tetramethyl ammonium bromide

46% deionized water

EXAMPLE 5 -

Compositions according to the present invention were prepared, coded as follows, and submitted to the Medical Entomology Centre (MEC) at the Department of Applied Biology, University of Cambridge, for testing. The compounds were:

1. "Original" = 0.8% w/v Cetyl trimethyl ammonium chloride

2. "Al khin -2" = 3.5% w/v lauryl pyridinium chloride

3. "Al khin -5" = 1.5% w/v lauryl dimethyl benzyl ammonium chloride

prepared as solutions the same way as described in Example 2 (excluding the Iso-propanol).

The following is an excerpt from the test report as received.

Materials

Lice were from the laboratory strain regularly maintained by MEC. They are rabbit adapted human clothing lice (Pediculushumanus humanus) and are physiologically indistinguishable from head lice (P. humanus capitis ). They are, however, larger and consequently able to withstand more rigorous environmental conditions than head lice.

Tests

Preliminary tests on these substances showed that lice that were engorged (recently fed) were more susceptible than lice fed the previous day. All lice used in these tests were fed not less than 24 hours prior to commencement.

Separate tests were conducted against adult and nymphal mobile stages and against eggs.

(a) Nymphs and adults

Lice were placed on a single disc of filter paper (Whatman No. 1) in a Petri dish. 5 ml. of the test solution was pipetted onto each such that the solution was evenly distributed and all lice were wetted.

The solution was poured off after 15 seconds. The volatile components were permitted to evaporate at room temperature for 20 minutes. The lice were then incubated at 27°C and 70% Relative Humidity (R.H.). Each test used approximately 20 lice and in triplicate. Untreated controls were also incubated.

(b) Eggs

Pieces of cotton corduroy cloth are used for lice to deposit their eggs during normal colony maintenance and changed at regular intervals. Eggs laid between 1 and 4 days previously were used in these tests.

The corduroy pads were immersed in the test solutions for 30 second. During that time they were turned once to ensure thorough wetting of the eggs. After removal they were blotted dry between two paper towels.

The dried corduroy pieces were inserted into bijou bottles and incubated at 27°C and 70% R.H. Each test was performed in triplicate and untreated controls were also set up.

RESULTS

(a) Tests against adults and nymphs were assessed after 24 hours and the % mortality recorded. The actual numbers of dead lice/total lice are in parentheses.

| GROUP | 1 | 2 | 3 |
|---|---|---|---|
| **Test substance** | | | |
| ADULTS | % Number | | |
| AL-KHIN-2 | 100 (20/20) | 100 (20/20) | 100 (25/25) |
| AL-KHIN-5 | 100 (24/24) | 100 (23/23) | 100 (20/20) |
| ORIGINAL | 100 (20/20) | 100 (20/20) | — |
| CONTROL | 0 (0/25) | 0 (0/22) | — |
| NYMPHS | | | |
| AL-KHIN-2 | 100 (19/29) | 100 (21/21) | 100 (19/19) |
| AL-KHIN-5 | 100 (20/20) | 100 (20/20) | 100 (12/12) |
| ORIGINAL | 100 (20/20) | 100 (20/20) | — |
| CONTROL | 0 (0/21) | 0 (0/20) | — |

These tests were checked by a further experiment using a 10 second exposure time and observed regularly during the day.

All the exposed lice became disorientated, making irregular and sometimes spasmodic movements and were frequently incapable of walking.

By 9 hours following exposure all were moribund and showed signs of severe disturbance of water balance. They were often stuck to the substrate filter paper by a glutinous secretion from the anus. They were checked again at 24 hours for % mortality and the actual numbers of dead lice/total lice were recorded.

| GROUP | ADULTS | NYMPHS |
|---|---|---|
| AL-KHIN-2 | 100 (20/20) | 100 (10/10) |
| AL-KHIN-5 | 90 (18/20) | 100 (10/10) |
| ORIGINAL | 31 (4/13) | 100 (10/10) |
| CONTROL | 0 (0/20) | 0 (0/10) |

(b) The average hatching period for rabbit fed louse eggs is 7-10 days. The eggs under test were observed at regular intervals up to and including the 14th day after treatment. All controls had hatched by the 10th day. An embryo is deemed to have survived the treatment if the larva develops to the stage of removing the egg cap even if it then dies before escape. The results are recorded as number hatched/total number of eggs.

| GROUP | 1 | 2 | 3 |
|---|---|---|---|
| Treatment | | | |
| AL-KHIN-2 | 1/120 | 0/95 | 0/150 |
| AL-KHIN-5 | 0/85 | 0/137 | 0/102 |
| ORIGINAL | 0/193 | 0/175 | — |
| CONTROL | 160/212 | 182/202 | — |

It is thus clearly seen that all three compounds kill all the lice totally - adults, nymphs and eggs altogether.

Example 6

More data, on the dependency between lice mortality and the concentration of the compound tested was obtained by separate independent experiments carried out by the inventor in the same laboratory of MEC. These tests were carried out in order to find the lowest concentration of each compound that still cause 100% mortality amongst lice. Only adults were tested. The figures are arithmetical at the average of three duplicates, 20 lice each. The lice were put on 9 cm filter paper and dipped in the solution for 30 seconds, then left to dry. The dry papers (with the lice) were incubated for 24 hours in 28°C. and final figures were as follows:

TABLE 2

| Compound | Concentration (%:w/v) | % Mortality |
|---|---|---|
| Lauryl pyridinium chloride | 0.95 | 66 |
| | 1.90 | 95 |
| | 3.80 | 100 |
| Lauryl dimethyl benzyl ammonium chloride | 0.35 | 90 |
| | 0.70 | 100 |
| | 1.50 | 100 |
| Cetyl trimethyl ammonium chloride | 0.6 | 36;95 |
| | 1.0 | 35 |
| | 2.0 | 87 |
| Tetra methyl ammonium bromide | 4.0 | 97 |

EXAMPLE 7 - Time curve of lice mortality:

The lice were exposed to one of the active compounds, lauryl dimethyl benzyl ammonium, in the same way as in Example 6. Mortality began 2 hours from exposure time. In 9 hours time almost all the lice had died.

TABLE 3

| Time Hours from exposure | Mortality % |
|---|---|
| 0 | 0 |
| 1 | 10 |
| 2 | 30 |
| 4 | 90 |
| 9 | 90 |
| 24 | 97 |

COMPARATIVE EXAMPLE 8 - Lice sensitivity to some other quaternary compounds and other compounds with similar structure.

Having found a series of quaternary ammonium compounds with very high pediculocide activity, it was decided to compare their activity with some other compounds which are similar in one of the following criterions:

1. Other quaternary ammonium compounds.

3 were chosen as follows: Alkyl dimethyl benzyl ammonium chloride, Lauryl trimethyl ammonium chloride and Alkyl oxyethyl ammonium phosphate.

2. Betains which were zwitterions of quaternary ammonium cations - Cocobetaine and Cocopropyl amido betains were used.

3. Antistatic agent which is not a quaternary compound

PEG 15 Tallow polyamine was used.

Tests were carried out as described before. Mortality was measured after 24 hours.

TABLE 4

| Material | Concentration used % | % Mortality |
|---|---|---|
| Alkyl dimethyl benzyl ammonium chloride | 1.5 | 50 |
| Lauryl trimethyl ammonium chloride | 1.5 | 65 |
| Alkyloxyethyl ammonium phosphate | 2.0 | 39 |
| Cocobetaine | 3.0 | 5 |
| Cocopropyl amido betaine | 3.0 | 30 |
| Alcohol + Water 1:1 (Control) | - | 0;20 |

The ingredients used in these tests was purchased from the following suppliers:

| | | |
|---|---|---|
| 1. | Cetyl trimethyl ammonium chloride (50%): | Utex-Akzo-chemie Co. Ltd. or Detex-Henkel Co. Ltd. |
| 2. | Lauryl pyridinium chloride (92%) | : Dehyquart C - -"-. |
| 3. | Lauryl dimethyl benzyl ammonium chloride (35%) | : Dehyquart LDB- -"-. |
| 4. | Lauryl trimethyl ammonium chloride(35%): | Dehyquart LT- -"-. |
| *5. | Alkyloxyethyl ammonium phosphate (50%) | : Dehyquart SP- -"-. |
| *6. | Alkyl dimethyl benzyl ammonium chloride | : Benzalkonium chloride-Teva Ltd. |
| 7. | Tetra methyl ammonium chloride (99%) | : Merck Co. Ltd. |
| 8. | Cocobetaine (30%) | : Dehytone AB-30 - Henkel Co. Ltd. |
| 9. | Cocopropyl amido betaine | : Tegobetaine HS-T.H. Goldschmidt AG. |

*Compound 5 is described by the manufacturer as a compound containing a mixture of alkyl groups with an average chain length of about ten carbon atoms and compound 6 is described by the manufacturer as a compound containing a mixture of capryl, lauryl, cetyl and meristyl as the alkyl group.

While all the quaternary compounds have some effect (the chloride more than the phosphate) all other compounds are, relatively, non active.

## Claims

1. An insecticidal composition characterized in that it comprises a quaternary ammonium compound of the general formula I

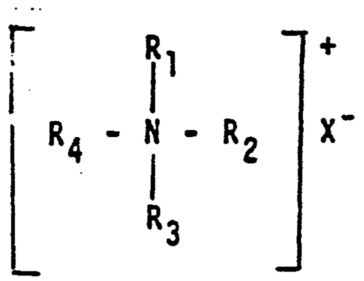

I

wherein each of $R_1$, $R_2$, $R_3$ and $R_4$ may be the same or different and are each individually selected from the group consisting of a $C_1$-$C_{20}$ alkyl group or a benzyl group or wherein $R_2$, $R_3$ and $R_4$ are combined with the N to which they are attached to form an optionally substituted pyridine or quinoline group and X is halogen.

2. An insecticidal composition according to claim 1 wherein at least one of $R_1$, $R_2$, $R_3$ and $R_4$ is an alkyl group.

3. An insecticidal composition according to claim 2 wherein at least one of $R_1$, $R_2$, $R_3$ and $R_4$ is an alkyl group having 8 to 18 carbon atoms.

4. An insecticidal composition according to claim 1 for pediculocidal use, wherein the quaternary ammonium compound is cosmetically acceptable and wherein $R_1$, $R_2$, $R_3$ and $R_4$ are each individually selected from the group consisting of methyl, $C_8$ to $C_{18}$ alkyl, or benzyl or wherein $R_2$, $R_3$ and $R_4$ are combined with the N to which they are attached to form an optionally substituted pyridine or quinoline group.

5. An insecticidal composition according to claim 1 for pediculocidal use, wherein at least one of $R_1$, $R_2$, $R_3$ or $R_4$ is selected from the group consisting of methyl, cetyl, lauryl or benzyl.

6. An insecticidal composition according to any preceding claim for pediculocidal use, wherein X is chlorine.

7. An insecticidal composition according to any preceding claim for pediculocidal use, comprising between 0.8% and 8.0% of the said quaternary ammonium compound as active ingredient therein, the remainder being a pharmaceutically acceptable excipient.

8. An insecticidal composition according to any preceding claim for pediculocidal use, comprising 0 to 70% of an alcohol solvent.

9. An insecticidal composition according to any preceding claim for pediculocidal use, wherein the quaternary ammonium compound is cetyl trimethyl ammonium chloride as active ingredient therein.

10. An insecticidal composition according to any of claims 1 to 8 for pediculocidal use, wherein the quatenary ammonium compound is lauryl pyridinium chloride as active ingredient therein.

11. An insecticidal composition according to any of claims 1 to 8 for pediculocidal use, wherein the quaternary ammonium compound is lauryl dimethyl benzyl ammonium chloride as active ingredient therein.

12. An insecticidal composition according to any of claims 4 to 11, in combination with instructions for the use thereof in the treatment of head and/or body and/or crab lice and/or lice eggs.

13. The use of a quaternary ammonium compound of the general formula I

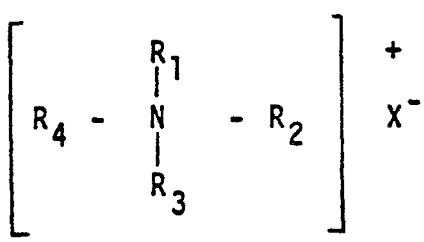

I

wherein each of $R_1$, $R_2$, $R_3$ and $R_4$ may be the same or different and are each individually selected from the group consisting of a $C_1$-$C_{20}$ alkyl group or a benzyl group or wherein $R_2$, $R_3$ and $R_4$ are combined with the N to which they are attached to form an optionally substituted pyridine or quinoline group and X is halogen, for the manufacture of a medicament for the therapeutic and/or prophylactic treatment of head and/or body and/or crab lice and/or lice eggs.

14. A process for producing an insecticidal composition according to any of claims 1 to 12, comprising combining the quaternary ammonium compound with a suitable excipient.

15. A method of using an insecticide, comprising applying to a locus an insecticidal composition according to any of claims 1 to 12.

European Patent Office

# EUROPEAN SEARCH REPORT

Application number

EP 85 30 9162

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-1 797 877 (W. MOORE)<br>* Whole document * | 1,2,4-6,13-15 | A 01 N 43/42<br>A 01 N 43/40<br>A 01 N 33/12 |
| A | US-A-4 439 427 (J.E. BERNSTEIN)<br>* Whole document * | 1,2,4-7,12-15 | |
| A | GB-A-1 538 768 (ALBERTO-CULVER)<br>* Page 1, lines 16-65; page 2, line 86 - page 3, line 47; page 3, line 60 - page 4, line 24; claims 1-3,12 * | 1-15 | |
| A | GB-A-1 593 601 (SOCIETE D'ETUDES DE RECHERCHES DE TRAVAUX D'ORGANISATION ET DE GESTION)<br>* Page 1, line 20 - page 2, line 4; claims * | 1,7,12-15 | TECHNICAL FIELDS SEARCHED (Int. Cl.4)<br>A 01 N |
| A | US-A-4 183 913 (E. ENDERS et al.)<br>* Column 1, lines 26-66; column 4, line 63 - column 5, line 35; column 6, lines 1-30; claims 1,2,6,7,12,14,15,18 * | 1-15 | |

The present search report has been drawn up for all claims

| Place of search | Date of completion of the search | Examiner |
|---|---|---|
| THE HAGUE | 07-05-1986 | FLETCHER A.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82